# FASCICULE DE BREVET EUROPEEN

(11) **EP 1 254 891 B1**
(45) Date de publication et mention de la délivrance du brevet: **11.10.2006**
(21) Numéro de dépôt: 02290904.8
(22) Date de dépôt: 11.04.2002
(51) Int. Cl.: C07C 219/08

(54) **Procédé de fabrication du (meth) acrylate de-2(dimethylamino)-1-(dimethylaminomethyl) ethyle**
Verfahren zur Herstellung der 2-(dimethylamino)-1-(dimethylaminomethyl)ethyle-(methyl)acrylat
Process of fabrication of 2-(dimethylamino)-1-(dimethylaminomethyl)ethyl-(methyl)acrylate

(30) Priorité: 26.04.2001 FR 0105609
(43) Date de publication de la demande: 06.11.2002
(73) Titulaire: Arkema France, 92800 Puteaux (FR)
(72) Inventeur: Riondel, Alain, 57600 Forbach (FR); Castellani, Fabrice, 57500 Saint-Avold (FR)
(74) Mandataire: Rieux, Michel

(56) Documents cités:
- EP-A- 0 281 718
- EP-A- 0 420 790
- EP-A- 0 663 386
- EP-A- 0 930 290
- DE-A- 3 048 020
- FR-A- 1 529 000
- FR-A- 2 027 225
- FR-A- 2 707 291
- DATABASE CA [en ligne] CHEMICAL ABSTRACTS SERVICE, COLUMBUS, OHIO, US; FUJIWARA, KEISUKE ET AL: "Preparation of tetrahydrobenzyl (meth) acrylate" retrieved from STN Database accession no. 124:30604 XP002183646 & JP 07 238057 A (DAICEL CHEM, JAPAN) 12 septembre 1995 (1995-09-12)
- DATABASE CA [en ligne] CHEMICAL ABSTRACTS SERVICE, COLUMBUS, OHIO, US; OKUTA, TATSUSHI ET AL: "Preparation of N-containing heterocyclylalkyl (meth) acrylates by transesterification" retrieved from STN Database accession no. 128:204893 XP002183647 & JP 10 072433 A (DAINIPPON INK AND CHEMICALS, INC., JAPAN) 17 mars 1998 (1998-03-17)

## Description

La présente invention porte sur un procédé de fabrication des composés de formule (I) : 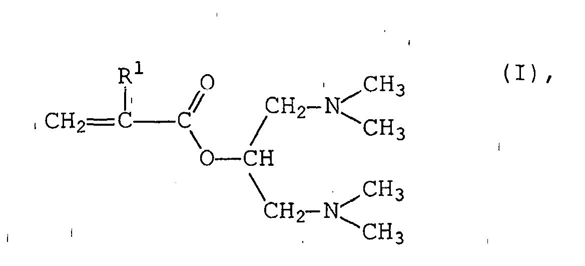 dans laquelle R¹ représente hydrogène ou un groupe méthyle,
également désignés dans ce qui suit par les abréviations S-ADAME (pour l'acrylate de 2-(diméthylamino)-1-(diméthylaminométhyl)éthyle) et S-MADAME (pour le méthacrylate de 2-(diméthylamino)-1-(diméthylaminométhyl)éthyle).

Le S-ADAME et le S-MADAME peuvent être quaternisés sur un ou deux azotes, pour donner, par exemple, avec l'agent quaternisant chlorure de benzyle, les composés respectivement : 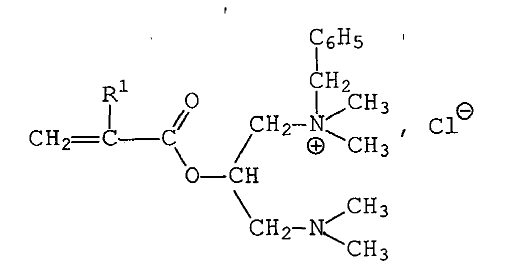 et 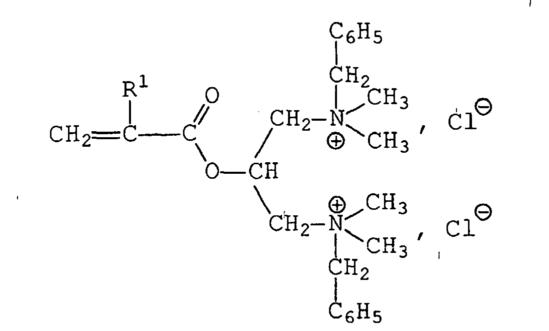 (S-(M)-ADAMQUAT 2BZ).

Les solutions aqueuses de sels quaternaires ainsi obtenues servent notamment à préparer des polymères destinés à servir de floculants cationiques dans le traitement des eaux.

On prépare le S(M)-ADAME en faisant réagir le 1, 3-bis-diméthylamino-2-propanol : 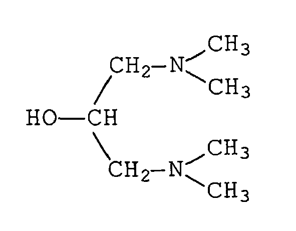 avec l'anhydride (méth)acrylique en présence de triéthylamine, avec un rapport molaire anhydride (méth)acrylique / 1,3-bis-diméthylamino-2-propanol de 0,5 à 2, à une température de 20 à 100°C, en particulier de 30 à 60°C, pendant un laps de temps de 2 à 10 heures, en présence d'au moins un stabilisant, tel que la phénothiazine, l'éther méthylique de l'hydroquinone, le 3,5-ditert.-butyl-4-hydroxytoluène et l'hydroquinone, et les mélanges de ces stabilisants, à raison de 200 à 3000 ppm par rapport à la charge. Dans la réaction avec l'anhydride (méth)acrylique, la triéthylamine sert à catalyser la réaction et à piéger l'acide (méth)acrylique formé sous forme de sel. Elle est utilisée généralement à raison de 1 à 2 équivalents molaires par rapport à l'anhydride (méth)acrylique.

Ce procédé n'est cependant pas facilement industrialisable car il génère des quantités importantes de rejets salins issus du piégeage de l'acide acrylique par la triéthylamine.

Egalement, M.A. KORSHONOV, F.N. BONDARYUK, V.S. MIKHLIN, Zh. Org. Khim. 1969 5(11) 1947-1952, ont décrit la synthèse du S-ADAME par transestérification, avec utilisation du titanate de tétrabutyle comme catalyseur. Toutefois, la pureté des produits obtenus par ce procédé est insuffisante.

Recherchant un procédé industrialisable pour la préparation des composés de formule (I) avec une plus grande pureté, la Société déposante a découvert que ce double but pouvait, de façon surprenante, être atteint si l'on utilise l'oxyde de dibutyl étain comme catalyseur dans la préparation des composés de formule (I) par transestérification.

La présente invention a donc pour objet un procédé de fabrication d'un composé de formule (I) : 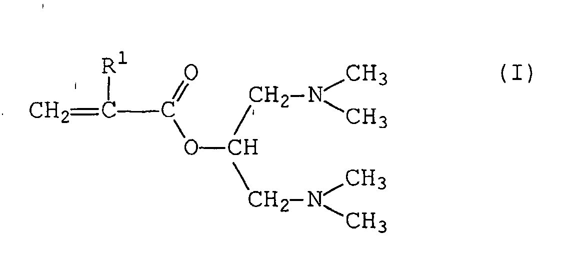 dans laquelle R¹ représente hydrogène ou un reste méthyle, suivant lequel on fait réagir un composé de formule (II) : 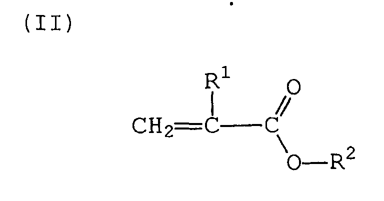 dans laquelle R² représente un reste alkyle linéaire en C₁-C₄, avec l'alcool de formule (III) : 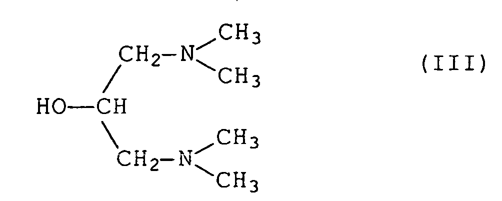 caractérisé par le fait que l'on conduit la réaction avec utilisation de l'oxyde de dibutyl étain comme catalyseur de transestérification.

Conformément à différentes caractéristiques préférentielles du procédé selon la présente invention :
- on utilise l'oxyde de dibutyl étain dans une quantité de 0,5% à 5% molaire, en particulier dans une quantité de 1% à 3% molaire par rapport à l'alcool de formule (III) ;
- on conduit la réaction à une température de 110 à 180°C, en particulier de 140 à 160°C ;
- on conduit la réaction avec un rapport molaire (méth)acrylate (II)/alcool (III) de 1,5 à 5, en particulier de 2 à 3 ;
- on utilise le (méth)acrylate de n-butyle comme composé (II) ;
- on conduit la réaction en présence de 500 à 3000 ppm d'au moins un stabilisant par rapport à la charge, le ou les stabilisants étant avantageusement choisis parmi la phénothiazine, le ditert.-butyl hydroxy toluène, l'éther méthylique de l'hydroquinone, la paraphénylènediamine et leurs mélanges en toutes proportions.

Les Exemples suivants illustrent la présente invention. Dans ces exemples, les parties et pourcentages indiqués sont en poids sauf indication contraire, et les abréviations suivantes ont été utilisées :
- ABU :: acrylate de n-butyle
- DBTO :: oxyde de dibutyl étain
- Zr(acac)₄ :: acétyl acétonate de zirconium
- S-ADAME :: acrylate de 2-(diméthylamino)-1-(diméthylaminométhyl)éthyle
- PTZ :: phénothiazine
- BHT :: ditertiobutyl hydroxy toluène

### EXEMPLE 1 :

Dans un réacteur en verre d'un litre, à double enveloppe, équipé d'une sonde de mesure de température, d'une canne plongeante pour l'introduction de l'air nécessaire à la stabilisation, d'un agitateur mécanique à vitesse variable, d'une colonne adiabatique de type Vigreux surmontée d'une tête de reflux, on charge :
- 154,74 g de 1,3-bis-diméthylamino-2-propanol ;
- 496,96 g d'ABU ; et
- 0,45 g d PTZ et 0,45 g de BHT comme stabilisants.

On porte le milieu à ébullition sous pression réduite (1,66 x 10⁴ Pa - 166 mbars) et on élimine l'eau par distillation azéotropique avec l'acrylate (il s'agit de l'eau contenue dans les réactifs et, plus particulièrement, dans le 1,3-bis-diméthylamino-2-propanol). Au cours de cette étape, on soutire environ 100 g d'un mélange ABU/H₂O constitué à plus de 99% d'ABU.

Ensuite, on introduit dans le réacteur 7,91 g de DBTO. La pression est fixée à 8,1 x 10⁴ Pa (810 mbars) et la température au cours de la réaction évolue de 145 à 159°C. On régule le soutirage de l'azéotrope ABU/BuOH par une température de consigne en tête de colonne (égale à 117°C).

La réaction est terminée lorsqu'on ne constate plus de formation de butanol en tête de colonne, autrement dit lorsque la température en tête de colonne est égale à celle d'ébullition de l'acrylate de butyle sous 8,1 x 10⁴ Pa (810 mbars).

Le S-ADAME est obtenu par distillation sous pression réduite du brut réactionnel. Sa pureté est de 99%.

### EXEMPLE 2 (comparatif) :

On conduit la même expérience qu'à l'Exemple 1, excepté que l'on remplace le DBTO par le Zr(acac)₄.

La pureté du S-ADAME obtenu par distillation sous pression réduite du brut réactionnel n'excède pas 98%.

### EXEMPLE 3 (comparatif) :

On conduit la même expérience qu'à l'Exemple 1, excepté que l'on remplace le DBTO par le titanate de n-butyle.

La pureté du S-ADAME obtenu par distillation sous pression réduite du brut réactionnel n'excède pas 98%.

## Revendications

1. Procédé de fabrication d'un composé de formule (I) : 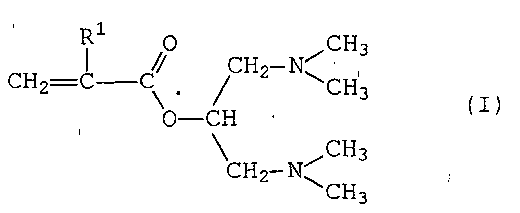 dans laquelle R¹ représente hydrogène ou un reste méthyle, suivant lequel on fait réagir un composé de formule (II) : 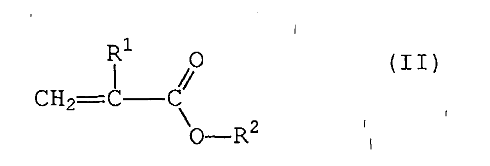 dans laquelle R² représente un reste alkyle linéaire en C₁-C₄, avec l'alcool de formule (III) : 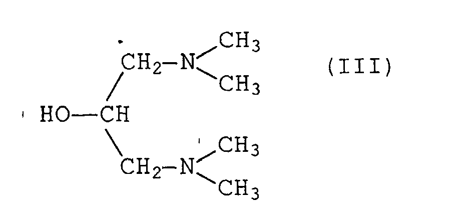 **caractérisé par le fait que** l'on conduit la réaction avec utilisation de l'oxyde de dibutyl étain comme catalyseur de transestérification.

2. Procédé selon la revendication 1, **caractérisé par le fait que** l'on utilise l'oxyde de dibutyl étain dans une quantité de 0,5% à 5% molaire par rapport à l'alcool de formule (III).

3. Procédé selon la revendication 2, **caractérisé par le fait que** l'on utilise l'oxyde de dibutyl étain dans une quantité de 1% à 3% molaire par rapport à l'alcool de formule (III).

4. Procédé selon l'une des revendications 1 à 3, **caractérisé par le fait que** l'on conduit la réaction à une température de 110 à 180°C.

5. Procédé selon la revendication 4, **caractérisé par le fait que** l'on conduit la réaction à une température de 140 à 160°C.

6. Procédé selon l'une des revendications 1 à 5, **caractérisé par le fait que** l'on conduit la réaction avec un rapport molaire (méth) acrylate (II)/alcool (III) de 1,5 à 5.

7. Procédé selon la revendication 5, **caractérisé par le fait que** l'on conduit la réaction avec un rapport molaire (méth)acrylate (II) / alcool (III) de 2 à 3.

8. Procédé selon l'une des revendications 1 à 7, **caractérisé par le fait que** l'on utilise le (méth)acrylate de n-butyle comme composé (II).

9. Procédé selon l'une des revendications 1 à 8, **caractérisé par le fait que** l'on conduit la réaction en présence de 500 à 3000 ppm d'au moins un stabilisant par rapport à la charge.

10. Procédé selon l'une des revendications 1 à 9, **caractérisé par le fait que** l'on utilise un stabilisant choisi parmi la phénothiazine, le ditert.-butyl hydroxy toluène, l'éther méthylique de l'hydroquinone, la paraphénylènediamine et leurs mélanges en toutes proportions.

## Claims

1. Process for preparing a compound of formula (I): 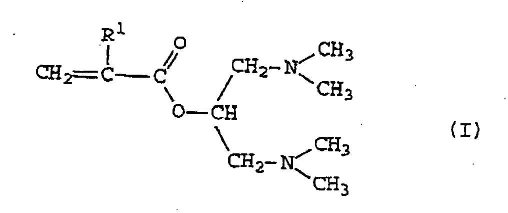 in which R¹ represents hydrogen or a methyl radical by reacting a compound of formula (II): 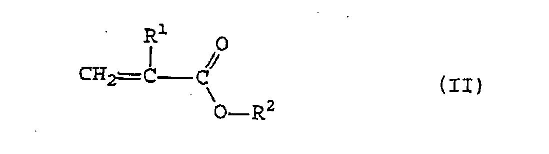 in which R² represents a linear C₁-C₄ alkyl radical with the alcohol of formula (III): 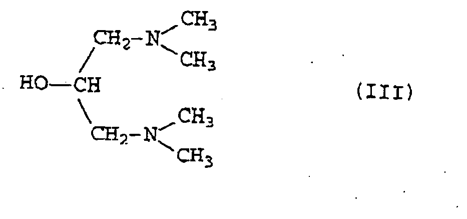 **characterized in that** the reaction is carried out using dibutyltin oxide as transesterification catalyst.

2. Process according to Claim 1, **characterized in that** dibutyltin oxide is used in an amount of from 0.5 to 5 mol% relative to the alcohol of formula (III).

3. Process according to Claim 2, **characterized in that** dibutyltin oxide is used in an amount of from 1 to 3 mol% relative to the alcohol of formula (III).

4. Process according to one of Claims 1 to 3, **characterized in that** the reaction is carried out at a temperature from 110 to 180°C.

5. Process according to Claim 4, **characterized in that** the reaction is carried out at a temperature from 140 to 160°C.

6. Process according to one of Claims 1 to 5, **characterized in that** the reaction is carried out with a (meth)acrylate (II)/alcohol (III) molar ratio of from 1.5 to 5.

7. Process according to Claim 5, **characterized in that** the reaction is carried out with a (meth)acrylate (II)/alcohol (III) molar ratio of from 2 to 3.

8. Process according to one of Claims 1 to 7, **characterized in that** n-butyl (meth)acrylate is used as compound (II).

9. Process according to one of Claims 1 to 8, **characterized in that** the reaction is carried out in the presence of from 500 to 3000 ppm of at least one stabilizer relative to the batch.

10. Process according to one of Claims 1 to 9, **characterized in that** a stabilizer is used selected from phenothiazine, di-tert-butylhydroxytoluene, hydroquinone methyl ether, para-phenylenediamine and mixtures thereof in any proportion.

## Patentansprüche

1. Verfahren zur Herstellung einer Verbindung der Formel (I): 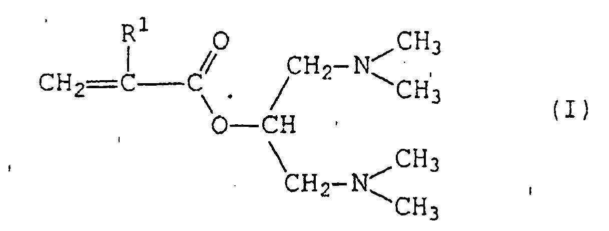 in welcher R¹ für Wasserstoff oder einen Methylrest steht, wobei verfahrensgemäß eine Verbindung der Formel (II): 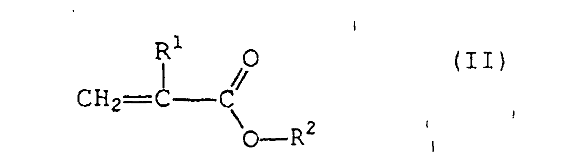 in welcher R² für einen geradkettigen Alkylrest von C₁-C₄ steht, mit einem Alkohol der Formel (III): 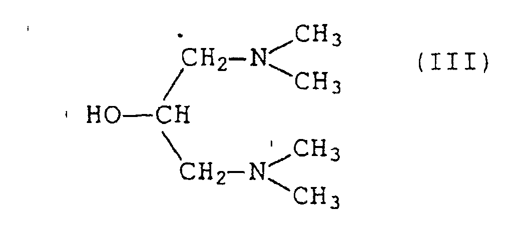 umgesetzt wird, **dadurch gekennzeichnet, dass** die Reaktion unter Verwendung von Dibutylzinnoxid als Umesterungskatalysator durchgeführt wird.

2. Verfahren nach Anspruch 1, **dadurch gekennzeichnet, dass** das Dibutylzinnoxid in einem molaren Mengenverhältnis von 0,5 % bis 5 % bezogen auf den Alkohol nach Formel (III) eingesetzt wird.

3. Verfahren nach Anspruch 2, **dadurch gekennzeichnet, dass** das Dibutylzinnoxid in einem molaren Mengenverhältnis von 1 % bis 3 % bezogen auf den Alkohol nach Formel (III) eingesetzt wird.

4. Verfahren nach einem der Ansprüche 1 bis 3, **dadurch gekennzeichnet, dass** die Reaktion bei einer Temperatur von 110 °C bis 180 °C durchgeführt wird.

5. Verfahren nach Anspruch 4, **dadurch gekennzeichnet, dass** die Reaktion bei einer Temperatur von 140 °C bis 160 °C durchgeführt wird.

6. Verfahren nach einem der Ansprüche 1 bis 5, **dadurch gekennzeichnet, dass** die Reaktion mit einem Molverhältnis von 1,5 bis 5 zwischen dem (Meth)acrylat (II) und dem Alkohol (III) durchgeführt wird.

7. Verfahren nach Anspruch 5, **dadurch gekennzeichnet, dass** die Reaktion mit einem Molverhältnis von 2 bis 3 zwischen dem (Meth)acrylat (II) und dem Alkohol (III) durchgeführt wird.

8. Verfahren nach einem der Ansprüche 1 bis 7, **dadurch gekennzeichnet, dass** n-Butyl(meth)acrylat als Verbindung (II) eingesetzt wird.

9. Verfahren nach einem der Ansprüche 1 bis 8, **dadurch gekennzeichnet, dass** die Reaktion in Gegenwart mindestens eines Stabilisators durchgeführt wird, wobei der Stabilisator im Verhältnis zu den Edukten in einer Menge von 500 bis 3000 ppm eingesetzt wird.

10. Verfahren nach einem der Ansprüche 1 bis 9, **dadurch gekennzeichnet, dass** ein Stabilisator verwendet wird, welcher aus den Verbindungen Phenothiazin, Ditert.-butylhydroxytoluol, Hydrochinonmethylether, para-Phenylendiamin sowie deren Mischungen in beliebigen Mengenverhältnissen gewählt wird.
